# EUROPEAN PATENT SPECIFICATION

(11) **EP 3 641 635 B1**
(45) Date of publication and mention of the grant of the patent: **09.12.2026**
(21) Application number: 18820591.8
(22) Date of filing: 31.05.2018
(51) Int. Cl.: G16H 50/20, G16H 50/70, G16H 30/40, G16H 40/67, A61B 5/00, A61B 5/055, A61B 6/00, A61B 6/02, A61B 6/03, A61B 6/50, A61B 8/00, A61B 8/08, G06N 3/082, G06N 3/098, G06N 3/0895, G06N 3/09, G06N 3/0464, G06V 10/82, G06V 10/774, A61B 6/46, G06N 3/063, G06N 3/045

(54) **DYNAMIC SELF-LEARNING MEDICAL IMAGE METHOD AND SYSTEM**
DYNAMISCHES SELBSTLERNENDES MEDIZINISCHES BILDVERFAHREN UND -SYSTEM
PROCÉDÉ ET SYSTÈME D'IMAGERIE MÉDICALE À AUTO-APPRENTISSAGE DYNAMIQUE

(30) Priority: 20.06.2017 US 201762522241 P
(43) Date of publication of application: 29.04.2020
(73) Proprietor: Hologic, Inc., Marlborough, MA 01752 (US)
(72) Inventor: CHUI, Haili, Fremont, CA 94539 (US); JING, Zhenxue, Chadds Ford, PA 19317 (US)
(74) Representative: Carpmaels & Ransford LLP
(86) International application number: PCT/US2018/035331
(87) International publication number: WO 2018/236565

(56) References cited:
- WO-A1-2015/066650
- JP-A- 2016 198 197
- KR-A- 20150 010 515
- KR-A- 20170 062 839
- US-A1- 2010 121 178
- US-A1- 2015 238 148
- US-A1- 2015 331 995
- AHMAD A AL SALLAB ET AL: "Self learning machines using Deep Networks", SOFT COMPUTING AND PATTERN RECOGNITION (SOCPAR), 2011 INTERNATIONAL CONFERENCE OF, IEEE, 14 October 2011 (2011-10-14), pages 21 - 26, XP032028187, ISBN: 978-1-4577-1195-4, DOI: 10.1109/SOCPAR.2011.6089108
- GHIASSI M ET AL: "A dynamic architecture for artificial neural networks", NEUROCOMPUTING, ELSEVIER, AMSTERDAM, NL, vol. 63, 20 August 2004 (2004-08-20), pages 397 - 413, XP027809659, ISSN: 0925-2312, [retrieved on 20050101], DOI: 10.1016/j.neucom.2004.03.014

## Description

### FIELD

The presently disclosed inventions relate generally to medical imaging techniques such as tomosynthesis, and more specifically to systems and methods for implementing a dynamic self-learning medical image network system. In particular, the presently disclosed inventions relate to interacting with, and observing user behavior pertaining to, one or more medical images at a plurality of nodes, in order to improve performance of the dynamic self-learning medical image network system.

### BACKGROUND

Medical imaging systems, e.g., tomosynthesis systems, CT scanning systems, MRI systems, mammography systems, etc., have been used for screening and diagnosing a variety of conditions. Doctors and other medical professionals often rely on medical images to diagnose various health conditions. Accurate readings of the medical images are contingent on the quality and clarity of the image, as well as the knowledge and expertise of the medical professional reviewing the image. Specifically, in order for the medical images to be helpful to medical professionals, they must clearly and accurately portray respective body parts to which the image pertains, such that the medical professional can efficiently make a prognosis with reasonable certainty. Radiologists (or other medical professionals) typically study thousands of such medical images, and are trained to detect, through time and practice, recurring patterns in the medical images that are indicative of abnormalities (or other objects of interest) in human tissue.

However, even with extensive training, the objects of interest to the medical professional can be difficult to identify within the medical image for a number of reasons. For example, the medical image may not provide sufficient clarity, or focus, such that a potential abnormality is overlooked. Or, the abnormality may be too small, or otherwise difficult to ascertain. In another example, the abnormality may not be a well-known abnormality, or one that a newly-trained medical professional has previously encountered. In some cases, human error may result in certain abnormalities being overlooked or misdiagnosed. Furthermore, the experience and knowledge from highly experienced practitioners is not easily transferred to others. As will be appreciated, such errors and omissions may have serious, and sometimes even fatal, consequences for patients. Also, existing medical imaging analysis systems, including in particular Computer Aided Detection (CAD) systems, must be frequently programmed and modelled with new information and/or updated analysis techniques, which is time consuming and resource-intensive. Thus, a medical imaging analysis system that minimizes reviewing errors and is automatically updated to provide the latest information and analysis techniques would be highly desirable.

Document WO 2015/066650 A1 discloses a medical information server. The medical information server receives a signal from a client device over a network, representing a first user interaction of a user with respect to first medical information displayed to a user. A user interaction analyzer invokes a first set of ECCD rules associated with the user based on the first user interaction to determine medical data categories that the user is likely interested in. The first set of ECCD rules is generated by an ECCD engine based on prior user interactions of the user. A data retrieval module accesses medical data servers corresponding to the medical data categories to retrieve medical data of the medical data categories. A view generator integrates the retrieved medical data to generate one or more views of second medical information and transmits the views of second medical information to a client device to be displayed on a display of the client device.

Document US 2015/331995 A1 discloses a medical information server. The medical information server receives a signal from a client device representing a first user interaction with first medical data associated with a first medical condition of a patient received from a first medical data server. A data retrieval module accesses a second medical data server to retrieve second medical data of the patient that is related to the first medical data. A data analysis module automatically performs a first analysis on image data of the first medical data to generate image quantitative result and a second analysis on the image quantitative result in view of other medical data of the patient to determine a likelihood of a second medical condition of the patient based on the analysis. A data integrator integrates the second medical data with an analysis result of the analysis to generate and transmit a view of medical information to the client device to be displayed therein.

### SUMMARY

In accordance with one aspect of the disclosed inventions, a method is provided for creating and using a dynamic self-learning medical image network system. In an exemplary embodiment, the method includes receiving, from a first node initial user interaction data pertaining to one or more user interactions with the one or more initially obtained medical images; training a deep learning algorithm based at least in part on the initial user interaction data received from the node; and wherein the initial user interaction data comprises an actual or estimated portion of at least one of the one or more medical images that was focused upon by at least one user, and/or a selection of one or more pixels associated with at least one of the one or more initially obtained medical images; transmitting an instance of the trained deep learning algorithm to the first node and/or to one or more additional nodes, wherein at each respective node to which the instance of the trained deep learning algorithm is transmitted, the trained deep learning algorithm is applied to respective one or more subsequently obtained medical images in order to obtain a result; wherein the result comprises recognizing one or more objects in the medical image; the method further comprising receiving, from the first node and/or one or more additional nodes, subsequent user interaction data pertaining to one or more subsequently obtained medical images, wherein the subsequent user interaction data is used to modify the trained deep learning algorithm.

By way of non-limiting examples, the initial user interaction data may include at least one annotation on at least one of the one or more initially obtained medical images, an actual or estimated amount of time one or more users spent viewing one or more of the initially obtained medical images, a description of a patient condition, and/or diagnostic findings that may be (without limitation) in a form of a written or a voice dictation report.

By way of non-limiting examples, the instance of the trained deep learning algorithm may be maintained at the first node and/or one or more additional nodes, and/or may run on a server accessed through a network.

By way of non-limiting examples, the result may include providing a recommendation pertaining to the medical image.

By way of non-limiting examples, the subsequent user interaction data is used to modify the trained deep learning algorithm if it is determined that the subsequent user interaction data satisfies a predetermined threshold confidence level indicating that the trained deep learning algorithm should be modified.

By way of non-limiting example, modification of the trained deep learning algorithm may include adding one or more layers to and/or changing the internal structure of, the layers in the trained deep learning algorithm.

In accordance with another aspect of the disclosed inventions, a dynamic self-learning medical image network system is provided, the system including a plurality of nodes, and a central brain server, wherein the central brain server is configured to receive initial user interaction data from one or more nodes of the plurality, wherein the initial user interaction data pertains to one or more user interactions with one or more initially obtained medical images, wherein the initial user interaction data comprises an actual or estimated portion of at least one of the one or more medical images that was focused upon by at least one user, and/or a selection of one or more pixels associated with at least one of the one or more initially obtained medical images, train a deep learning algorithm based at least in part on the initial user interaction data received from the node, and transmit an instance of the trained deep learning algorithm to each node of the plurality, and wherein each node of the plurality is configured to apply the instance of the trained deep learning algorithm to one or more subsequently obtained medical images in order to obtain a result; wherein the result comprises recognizing one or more objects in the medical image; wherein the central brain server is configured to receive subsequent user interaction data from one or more nodes of the plurality pertaining to one or more subsequently obtained medical images, and to modify the trained deep learning algorithm if the subsequent user interaction data satisfies a predetermined threshold confidence level indicating that the trained deep learning algorithm should be modified.

In an exemplary embodiment, each node of the plurality is configured to maintain an instance of the trained deep learning algorithm.

By way of non-limiting examples, the initial user interaction data received by the central brain server may include at least one annotation on at least one of the one or more initially obtained medical images, an actual or estimated amount of time one or more users spent viewing one or more of the initially obtained medical images, a description of a patient condition, and/or diagnostic findings that may be (without limitation) in a form of a written or a voice dictation report.

By way of non-limiting examples, the result may include providing a recommendation pertaining to the medical image.

By way of non-limiting example, central brain server may modify the trained deep learning algorithm by adding one or more layers to the trained deep learning algorithm.

These and other aspects and embodiments of the disclosed inventions are described in more detail below, in conjunction with the accompanying figures.

### BRIEF DESCRIPTION OF THE FIGURES

The drawings illustrate the design and utility of embodiments of the disclosed inventions, in which similar elements are referred to by common reference numerals. These drawings are not necessarily drawn to scale. In order to better appreciate how the above-recited and other advantages and objects are obtained, a more particular description of the embodiments will be rendered, which are illustrated in the accompanying drawings. These drawings depict only typical embodiments of the disclosed inventions and are not therefore to be considered limiting of its scope.
FIG. 1 is a block diagram illustrating the dynamic self-learning medical image network system constructed in accordance with embodiments of the disclosed inventions;
FIG. 2 is a sequence diagram illustrating the flow of information between a user and a central brain network constructed in accordance with embodiments of the disclosed inventions;
FIG. 3 illustrates one embodiment of recording user interactions in a dynamic self-learning medical image network system constructed in accordance with embodiments of the disclosed inventions;
FIGS. 4A and 4B illustrate an exemplary flow diagram depicting various steps to modify (and thereby improve) the dynamic self-learning medical image network system over time; and
FIGS. 5A to 5H illustrate an exemplary process flow in accordance with embodiments of the disclosed inventions.

### DETAILED DESCRIPTION OF THE ILLUSTRATED EMBODIMENTS

All numeric values are herein assumed to be modified by the terms "about" or "approximately," whether or not explicitly indicated, wherein the terms "about" and "approximately" generally refer to a range of numbers that one of skill in the art would consider equivalent to the recited value (i.e., having the same function or result). In some instances, the terms "about" and "approximately" may include numbers that are rounded to the nearest significant figure. The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. In describing the depicted embodiments of the disclosed inventions illustrated in the accompanying figures, specific terminology is employed in this patent specification for the sake of clarity and ease of description. However, the disclosure of this specification is not intended to be limited to the specific terminology so selected, and it is to be understood that each specific element includes all technical equivalents that operate in a similar manner. It is to be further understood that the various elements and/or features of different illustrative embodiments may be combined with each other and/or substituted for each other wherever possible within the scope of this specification, including without limitation the accompanying figures and the appended claims.

Various embodiments of the disclosed inventions are described hereinafter with reference to the figures. It should be noted that the figures are not drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the disclosed inventions, which is defined only by the appended claims and their equivalents. In addition, an illustrated embodiment of the disclosed inventions needs not have all the aspects or advantages shown. For example, an aspect or an advantage described in conjunction with a particular embodiment of the disclosed inventions is not necessarily limited to that embodiment and can be practiced in any other embodiments even if not so illustrated.

### Introduction

This patent specification and the accompanying figures describe and illustrate a dynamic self-learning medical image network system that utilizes deep learning techniques to observe user interactions with medical images at a plurality of nodes. These user interactions are compiled, analyzed and optimized using a central brain network that advantageously trains one or more deep learning algorithms/network to continuously improve readings of medical images over time. As will be discussed throughout the specification, the dynamic self-learning medical image network system advantageously leverages expertise gained from users who are highly trained in reading medical images and decodes this information in deep learning algorithms, such that the system is constantly learning and improving analysis of medical images over time, in an effort to ultimate emulate the skill and intuition of a human expert.

Accurate reading of medical images, such as without limitation MRI scans, CT scans, tomosynthesis slices, X-rays, etc., is often contingent on the skill and technical expertise of medical personnel evaluating them. This expertise is gained through time, practice and intuition that is developed as a result of viewing a very large number of medical images. However, since there is a wide range in the respective skill, efficiency and expertise of the medical personnel charged with reading the images, the quality and accuracy of image-based screening and diagnosis can vary greatly. Further, even in the case of highly skilled medical professionals (e.g., radiologists), human error sometimes causes missed diagnoses, inaccurate readings and/or false positives. Of course, such errors, although easy to make, adversely affect the quality of healthcare delivered to patients, and can cause great stress and anxiety to everyone involved.

Although there are many medical image analysis software (CAD) programs available in the market today, they are static modelling programs that have to be programmed and trained prior to implementation, and as such can quickly become outdated. Re-programming to update the system is costly and time intensive, and still tends to lag behind research or new findings, even if routinely performed.

### Advantages of the dynamic self-learning medical image network

One approach to globally improve the quality of medical image screening and diagnosis is to aid the reader of the medical images (also referred to herein as the system "user") by implementing a dynamic self-learning medical imaging network system that interacts with a large number of users (e.g., expert radiologists), analyzes many types of medical data, e.g., medical images, patient information data, patient medical records, etc., and automatically learns to interpret medical images and data to identify patterns (which may be image patterns or non-image patterns) that are symptomatic of abnormalities.

A dynamic self-learning medical image network system may be defined as a system that is continuously updating and/or adding layers to one or more deep neural networks, without necessarily requiring manual re-programming. In other words, rather than relying (and waiting) on presumed (if not actual) experts that understand trends in radiology or image analysis to create a new static program or update an existing one, the dynamic self-learning system is continually learning from the actual (and truly expert) users, by analyzing the user interactions with the system and periodically adding layers to the deep neural networks based on this analysis. This approach has several advantages. First, by studying user interactions with a very large number of medical images, the accuracy of the system in detecting such patterns not only improves over time, but also contemporaneously with the user. For example, if users are beginning to identify a previously unknown mass as being an abnormality, the dynamic self-learning medical image network system obtains this information in real-time, and is able to start identifying such abnormalities on its own, without necessarily being re-programmed by a system administrator. Additionally, the system learns patterns not just from a limited training dataset, but from a very large, and indeed ever-growing dataset. For example, thousands of radiologists may mark a particular type of breast mass as a spiculated mass (e.g., a type of breast cancer lesion). Having digital data of these diagnoses allows the system to study the patterns of the images that have been marked by the users as constituting a spiculated mass. Thus, the dynamic self-learning system described and depicted herein may strategically leverage the expertise of tens (or hundreds) of thousands of users in real-time to develop a highly accurate image recognition system.

Further, the dynamic self-learning medical image network system described and depicted herein allows users to rely on the system (and other users) in reaching a diagnosis. For example, a particular doctor (or group of doctors) may have special expertise when dealing with a rare form of abnormality. Or the abnormality may have only been recently detected by a small group of users in a particular part of the world. By leveraging knowledge that may only be available in one local community, the dynamic self-learning system may assist users in other communities worldwide by automatically detecting the heretofore unknown or little-known condition. Thus, information may spread far more swiftly with such an integrated image recognition system that is connected to users having varying skills, expertise, geography and patient type.

Moreover, by tracking interactions of a large number of users, the dynamic self-learning system may learn that certain users are especially skilled or knowledgeable, e.g., based on the rate of accurate screenings, and interactions with such users may be weighted higher than the average user of the medical image system. Conversely, if it is determined that certain users are less skilled, the dynamic self-learning system may avoid or minimize learnings from such user interactions, but may instead assist such users with information gained from users that have greater expertise. Thus, the dynamic self-learning medical image network system may actively assist users in improving readings of medical images.

The dynamic self-learning medical image network system may be trained to observe a set of user interactions pertaining to one or more medical images, and pool data received from a plurality of users in order to learn aspects pertaining to medical image and user interface interaction. For example, one learning aspect may relate to image analysis itself, and the dynamic self-learning medical image network system may observe user behavior related to medical image to detect recurring patterns in medical images that are indicative of abnormalities. Another example of a learning aspect may relate to user experience, and how to improve a set of task flows such that a user is provided an optimal amount of information to quickly and efficiently reach a prognosis. Yet another example of a learning aspect may relate to learning medical history associated with a patient and presenting that information to the medical professional to provide a more comprehensive diagnosis.

Although the present specification focusses on learning aspects related to image analysis and diagnosis, it should be appreciated and understood that the dynamic self-learning medical image network system may observe a myriad of user interactions to improve various aspects of the system. By constantly learning through user interactions with medical images in many locations, the dynamic self-learning medical image network system detects (or aids medical professionals in detecting) abnormalities, thereby increasing the efficiency and accuracy of screening and diagnoses performed using medical images. Ultimately, by learning details pertaining to highly skilled user decisions regarding medical images (also referred to herein as "medical image process flow"), the dynamic self-learning medical image network system becomes increasingly accurate and reliable over time, such that it may attempt to emulate the skill (or indeed even the subconscious intuition) of such highly skilled users.

The present specification focuses on implementing a dynamic self-learning medical image network system using deep machine learning algorithms (e.g., neural networks) to learn from users and data. It is envisioned that the system may learn independently with little need for manual programming or programmer input. In particular, in recent years, there have been major improvements in the field of machine learning using deep learning systems to recognize images and to understand natural languages. In many applications, the machine learning algorithm can be trained to learn to perform tasks at similar performance levels as a human. By building upon such machine learning algorithms, an "expert-level" self-learning medical image network system may be created that can be used to extract patterns and detect trends that may be too complex to be detected through traditional computer technologies but easily detected by an expert human user. Thus, the dynamic self-learning medical image network system may become an "expert" assistant to the medical professional reading the medical image. Other suitable means to achieve such a complex learning may be similarly implemented without limitation.

### System Overview (including interaction between various nodes and the central brain)

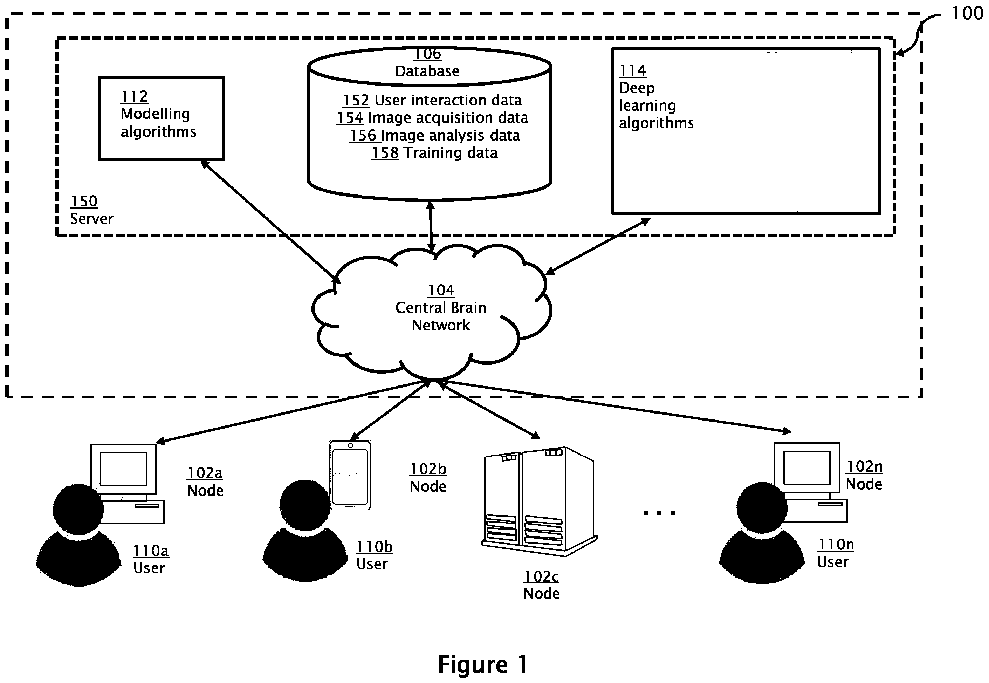

FIG. 1 illustrates an overview of the dynamic self-learning medical image network system 100 which incorporates image generation, image analysis and network technology. It should be understood that while FIG. 1 illustrates a particular embodiment with certain processes taking place in a particular serial order or in parallel, the claims and various other embodiments described herein are not limited to any particular order, unless so specified. More particularly, the dynamic self-learning medical image network system 100 includes a plurality of nodes 102 (e.g., 102a, 102b, 102c... 102n) that interact with a central brain network 104. In one or more embodiments, the nodes 102 refer to a computing system that may or may not interact with a user. As shown in FIG. 1, nodes 102a and 102b interact with the users, but node 102c does not. In some embodiments, the nodes 102 may refer to a point of contact between the dynamic self-learning medical image network system 100 and a user 110 (e.g., 110a, 110b... 110n). The nodes 102 may be any type of computing device including a processor and/or display system (e.g., personal computer, specialized imaging system, smartphone, a tablet, an image acquisition device, e.g., MRI, CT, tomosynthesis system), an image review workstation, a virtual reality device, desktop computer, web portal, etc. In some embodiments, the respective nodes may each be some other type of machine-human user interface.

Each node 102 may be implemented on a picture archiving and communications system (PACS). For example, a respective node 102 may be a dedicated medical image viewing workstation allowing users 110 to perform a variety of specialized image-related tasks. The nodes 102 may include one or more network interfaces for communicating with other devices through a network. The nodes 102 may include other input/output devices that enable user interaction with the node, such as a display, keyboard, mouse, audio speakers, and the like. It should be appreciated that the node may function with or without the user. For example, in some embodiments, the node 102 may be an intelligent workstation that mimics or attempts to make decisions like a human.

In some embodiments, a particular node 102 may represent an algorithm or data server that may be running data mining algorithms. In other embodiments, the node 102 may be a computing device that gathers data. In some embodiments, the node 102 may be a PACS machine that gathers images. In still other embodiments, the node 102 may simply be software that is running on a hospital computer system. Thus, it should be appreciated that not all nodes 102 necessarily interact with users, and some nodes 102 may simply gather data, or learn from data itself while other nodes 102 also provide and receive user interaction.

A user 110 accessing the dynamic self-learning medical image network system 100 is typically a medical professional (e.g., general doctor, radiologist, medical technician), but it should be appreciated that the dynamic self-learning medical image network system 100 is capable of interacting with any user (e.g., non-medical professionals, patients, etc.) no user at all. For purposes of illustration, the remainder of this specification focusses on medical professional users, but this should not be understood as limiting the applicability of the dynamic self-learning medical image network system.

The central brain network 104 may be any type of network known in the art, such as the Internet, or any cloud computing network. In one or more embodiments, the nodes 102 may communicatively couple to the central brain network in any manner, such as by a global or local wired or wireless connection (e.g., LAN, WAN, intranet, etc.). In one or more embodiments, the central brain network 104 may be communicatively coupled to one or more servers 150 or other machines which may include one or more processing units and/or computer readable media. In one or more embodiments, the central brain network 104 may reside (and be maintained) on one or more physical computing devices, or it may reside on a virtual cloud computing network. In its simplest form, it can be a very powerful central computing device communicatively coupled to a plurality of nodes. In a more complex form, the central brain network 104 may take a distributed form and reside over a number of physical or virtual computing devices.

More particularly, the server(s) 150 may house and/or host a plurality of computing components that together process data received from the plurality of nodes 102, store data, and provide outputs that are sent back to the nodes 102. As will be discussed in further detail below, the data may pertain to medical images being viewed and interacted with at the plurality of nodes 102. This data may be processed, analyzed, stored and updated through the various computing components of the server 150, and updated data may be sent back to the nodes 102 through the central brain network 104. In one or more embodiments, the server 150 may be a single powerful server. In another embodiment, a distributed system having multiple servers performing sub-sections of the computing tasks is envisioned. The server(s) 150 may be located in one geographical location, or may be located at different locations throughout the world. In one or more embodiments, an instance of the server 150 is operable to run at the node 102, such that an instance of the dynamic self-learning medical image network system runs on the node itself. The server(s) 150 may refer to local servers or remote servers.

In one or more embodiments, the server(s) 150 include one or more database(s) 106 that store all or portion of the data related to the dynamic self-learning medical image network system 100. The database(s) 106 may be the central data store providing long-term storage for all data, or it may be a limited-purpose data store for a specific area. The database(s) 106 make data accessible to the central brain network 104. The server(s) 150 may include computing components that are operable to retrieve data from the one or more databases and supply it to the central brain network 104 through a server-network interface. Although depicted as a single database 106 in FIG. 1, it should be appreciated that any number of local or remote databases may be part of the dynamic self-learning medical image network system 100. In one or more embodiments, the database 106 may store image acquisition data 154 that may be displayed to the users 110 at the various nodes 102. The database 106 may also store image analysis data 156, or data related to analysis of the various medical images. In one or more embodiments, training data 158 may also be used to train the dynamic self-learning medical image network system 100.

Medical images typically refer to digital representations of one or more objects (e.g., parts or portions of a patient's body, such as breasts). The digital representations may be modified or manipulated in order to identify or enhance certain features of the image. Such manipulations are virtual manipulations accomplished through the various computing components of the server(s) 150.

The analysis data may originate from the users or may be computer-generated analysis data. The database 106 may also store a set of user interaction data 152. The user interaction data 152 may be any data collected from the plurality of users 110. For example, the user interaction data 152 may be detected patterns indicative of known abnormalities, and also may contain feature values (e.g., coordinates, grayscale values, contrast values, etc.) related to abnormalities (e.g., cysts, tumors, abnormal masses, spiculated masses, calcifications, etc.). In one or more embodiments, the database(s) 106 include a constantly updated/modified learning library that improves over time based on the collected user interaction data. In one or more embodiments, the learning library may store a set of rules and/or models that may be used by the server(s) for image analysis.

In one or more embodiments, the server(s) 150 include one or more algorithms 112 that ingest a set of data pertaining to user interaction with a plurality of images, and create data models that may be used to detect patterns indicative of abnormalities in the medical images. The algorithms 112 may relate to image analysis, image display, or any other processes related to data that is present and interacted with at the nodes 102. Although the present specification focusses on image analysis algorithms, it should be appreciated that any type of algorithm 112 may be created and stored.

In one or more embodiments, the server(s) 150 include one or more deep learning algorithms or deep neural networks 114 that are trained on medical image data to learn complex image patterns and detect anatomical landmarks. A deep learning algorithm may refer to a deep neural network comprising various layers of information. Deep learning algorithms 114 contain multiple layers of learned features and/or variables between the input data and the output data. Deep learning algorithms 114 or deep neural networks may be implemented with many layers that are built on top of each other, such that complex deep learning algorithms comprise several deep layers, e.g., tens, hundreds, or even thousands of layers, that are continuously added as the system learns more information. A deep neural network may be differentiated with typical neural networks that tend to be "shallow" neural networks comprising only a few layers, e.g., only three or four layers. Thus, deep neural networks tend to be far more complex than shallow neural networks.

The deep learning algorithms 114 may be trained to detect patterns or a localization (e.g., pixel or voxel coordinates) in a medical image. In one or more embodiments, deep learning algorithms may be trained based on a plurality of training images. For example, the deep learning algorithms 114 may be trained using the user interaction data stored in the database(s) 106. The training images may be 2D or 3D medical images acquired through any type of medical image modality (e.g., tomosynthesis, mammography, CT, MRI, ultrasound, etc.). It should be appreciated that at least a subset of the training images may be annotated with the location of the respective anatomical object or landmark. For example, the user interaction data stored in the database(s) 106 may contain annotations (collected from the plurality of users 110) in the medical images identifying a desired object (e.g., type of mass, abnormality, type of tissue, etc.).

In some embodiments, the training images may be non-annotated but may tag objects in some other fashion. The deep learning algorithms 114 adapt and modify over time so as to to improve their accuracy, efficiency and/or other performance criteria with a larger and larger number of user interaction data, thereby detecting and localizing desired anatomical landmarks or objects in the medical images with greater precision. Although there may be many possible implementations of deep learning algorithms to recognize abnormalities in medical images, one possible implementation approach includes one or more deep learning algorithms that calculate a probability that a targeted anatomical object is located at a particular pixel or voxel. In another possible implementation, the deep learning algorithms may calculate a difference vector from a particular voxel to a predicted location of the target object.

Thus, deep learning algorithms 114 may be utilized in one of many possible implementations and be trained, using user interaction data, to detect target objects or abnormalities in the medical images. By collecting vast amounts of user interaction data, the deep learning algorithms may be trained to become increasingly precise over time. It should be appreciated that that the deep neural networks or deep learning algorithms are updated dynamically, in contrast to static neural networks that are used to provide results based on a pre-programmed algorithm. Static neural networks do not adapt to new information, whereas the deep neural network system described and depicted herein "learns" from various user interactions, and updates, modifies and/or adds layers to the deep learning neural network(s). The term "dynamic," in this context, refers to a deep-learning system that is continually updated or modified automatically without specific need for re-programming. In particular, the deep neural network system preferably automatically updates the respective deep neural networks by adding one or more layers and/or changing the structure of one or more existing layers, once it is understood by the system that additional complexity pertaining to a pattern is required or otherwise useful. This is an important distinction from existing deep neural network algorithms, which (once trained) merely modify the respective layer weighting parameters without otherwise changing or adding layers.

There may be many ways in which to add layers to the deep neural network and/or update the deep neural network contemporaneously while a plurality of users interacts with the system. For example, the system might pool together data from a large number of users to determine a threshold level of confidence before adding a layer of complexity to the existing deep learning algorithm. The threshold levels may be predetermined, in one or more embodiment. Or, in another embodiment, the threshold level may refer to a particular number of users corroborating a particular detail. In yet another embodiment, programmer input may be requested prior to adding another layer. Once a particular confidence level is achieved, one or more layers may be added, or the neural network may be modified to conform to the newly "learned" data.

### Example implementation of dynamic self-learning medical image network system

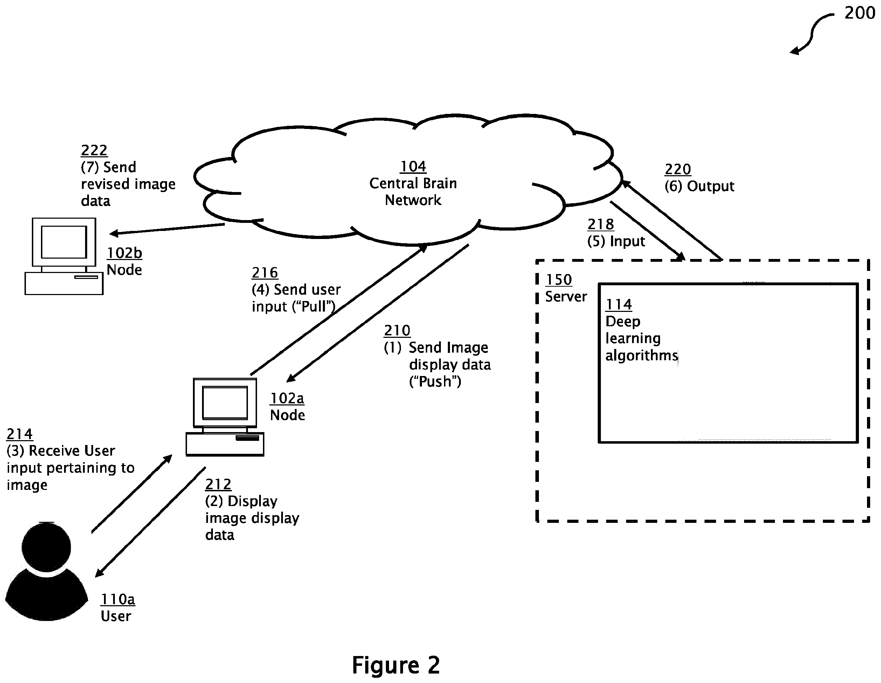

Referring now to FIG. 2, a system diagram showing an example sequence of interaction with the various components of the dynamic self-learning medical image network system is illustrated. As discussed in detail with reference to FIG. 1, at 210, a set of image display data may be sent to the node 102a through the central brain network 104. In one or more embodiments, an instance of an initial deep learning algorithm/network may also be sent to the node to perform image analysis. The initial deep learning algorithm may be trained at the central brain network using existing data, and known images, or training data. An instance of the initial deep learning algorithm may be pushed to one or more nodes of the dynamic self-learning image network system. In one or more embodiments, the image display data may be medical images (e.g., tomosynthesis image slices of a patient's breast tissue). At 212, the user 110a (if the node interacts with a user) interacts with the node 102a, and is able to view the image display data. In one or more embodiments, the initial deep learning algorithm may be run on the medical images, and one or more results may be provided to the user 110a.

At 214, the user may interact with the medical image (e.g., annotate the medical image, zoom into particular aspects of the medical image, focus on a particular slice if there are multiple slices, etc.) in one or more ways. For example, in viewing a particular tomosynthesis slice, the user may mark a particular set of pixels of the image and annotate that part of the image as indicative of a spiculated mass lesion. This user interaction may be recorded at the node 102a. The various types of possible user interactions will be discussed further below. Or, the user 110a may concur or reject the analysis provided by the deep learning algorithm. User interactions are collected in addition to the provided analysis such that the dynamic self-learning image network system is constantly collecting user interaction information even if a current instance of the (initially trained) deep-learning algorithm is run on one or more medical images.

At 216, the user interaction data recorded at the node 102a (e.g., annotations, marked pixels, audio and/or video recordings, etc.) may be sent to the central brain network 104. At 218, the user interaction data (input) is received at the server 15 through the central brain network 104. In one or more embodiments, the user interaction data may be used as additional training data on the deep learning algorithms 114. As discussed above, the deep learning algorithms 114 may consume the user interaction data to learn patterns or features associated with a spiculated mass as highlighted by the user. This interaction (along with other user interaction data collected from all the other nodes of the dynamic self-learning medical image network system) allows the deep learning algorithms 114 to automatically recognize spiculated masses (and other abnormalities learned by the deep learning algorithms 114) based on digital information associated with the medical image. The modified (improved) deep learning algorithm information may be stored at one or more databases at the server 150.

In practice, not every new user interaction data will be used (or be useful) to modify (with the goal of improving) an existing deep learning algorithm. The example discussed above is simplified for illustrative purposes. Rather, the newly collected user interaction data may be used to run one or more data-mining/un-supervised learning algorithm to form a new understanding of the complexity of the new data. Once this complexity reaches a certain threshold level, more layers may be added to the deep learning algorithm to create an improved/updated deep learning algorithm that is more complex and contains insights from more data. The improved/updated deep learning algorithm may be further trained on more preliminary/training data before it is pushed back to various nodes of the dynamic self-learning medical image network system.

At 220, the improved/updated deep learning algorithms are communicated to the various nodes through the central brain network 104. At 222, an instance (or partial instance) of the improved/updated trained deep learning algorithms may be transmitted to any node (e.g., 102b), which may then be used to provide feedback and/or provide image analysis at the node 102b. The improved/updated trained deep learning algorithms may run on the node 102b in order to automatically recognize spiculated masses (or other abnormalities) found in other medical images residing at the node 102b. For example, this improved/updated deep learning algorithm information may be used on another medical image viewed by a user at node 102b. The node 102b (leveraging the improved deep learning algorithms) may automatically mark portions of the other medical image if the system determines that a particular area of the medical image contains a spiculated mass object. This information may be displayed to the user at node 102b, wherein the user may confirm or reject the automatically detected object. This interaction may also be recorded and sent back to the server 150 through the central brain network 104 to further improve the deep learning algorithms 114. Thus, it is envisioned that the deep learning algorithms becomes increasingly skilled at recognizing objects found in the medical images over time.

As discussed above, user interaction data collected at the various nodes 102 of the dynamic self-learning medical image network system 100 is continuously used, in regular intervals, to improve the deep learning algorithms 114. Thus, the current invention(s) describe a dynamic self-learning medical image network system that is constantly learning in real-time as it is being deployed at various nodes. In contrast to static neural networks that have to be manually programmed or re-programmed periodically, the dynamic self-learning medical image network system is continuously adding more layers (or otherwise modifying itself) to the deep learning algorithms without necessitating reprogramming to create a new neural network. When new data is learned that adds to the complexity of the existing deep learning algorithms, new layers are automatically added to the deep learning algorithm, and pushed to the various nodes.

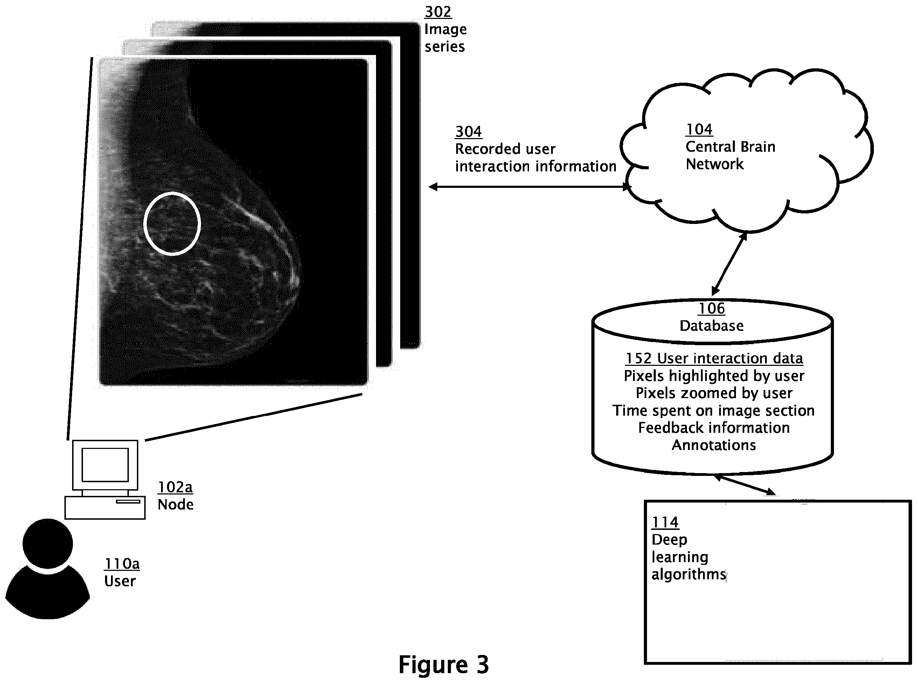

Referring now to FIG. 3, an overview of how various types of user interactions with medical images is utilized in the dynamic self-learning medical image network system is illustrated. As shown in FIG. 3, the user 110a at the node 102a may be presented with a series of medical images 302. For example, the series of medical images 302 may be tomosynthesis slices representative of a patient's breast tissue. The user 110a may interact with the series of images 302 in a number of ways. For example, the user 110a may zoom in to view particular tomosynthesis slices. Also, the user 110a may concentrate on just a subset of the image slices, while ignoring the rest. Additionally, the user 110a may expressly mark a portion of the digital image, and annotate it. Furthermore, the user 110a may create a video or audio recording of the user's diagnosis.

In one or more embodiments, the user 110a may immediately focus one or more of the image slices 302 to focus on. For example, the user 110a may spend most of the time focused on image slice x. The dynamic self-learning medical image network system 100 may record this interaction to determine whether there are any patterns in what image slice(s) provide the most valuable information. In one or more embodiments, the dynamic self-learning medical image network system 100 may track the actual time and/or an estimate of an amount of time spent on a particular image or images (slice or slices). This information may be coupled with other collected user interaction data to learn what parts of an image deck are most important when analyzing images.

In another example, the dynamic self-learning medical image network system may ask the user 110a to mark or otherwise annotate a particular image with information regarding the medical image. In one possible implementation, a set of users may be selected to train the dynamic self-learning system. These users may be asked to annotate various medical images with many types of abnormalities. The system may thereafter pool images belonging to or associated with a type of abnormality, and then identify ("learn") patterns emerging from a relatively large image dataset.

Other types of user interactions that may be recorded include pixels of the medical image that may be highlighted or zoomed by the user. The dynamic self-learning medical image network system may record the time spent on an image or set of images. Similarly, any number of such interactions may be received and recorded.

Any or all of these user interactions 304 may be sent to the server 150 through the central brain network 104, and further stored in the learning database 106. The learning database 106 may comprise user interaction data 152 received from thousands or millions of nodes. As shown in FIG. 3, the user interaction data 152 may comprise pixels highlighted by the user, areas (e.g., defined by pixels) of an image that were focused upon (e.g., "zoomed in" on) by the user, actual or estimated time spent on image portions, feedback on images, annotations, or any other type of user interaction. Similarly, other types of user interactions (e.g., 154, 156 and 158) may be similarly stored, although omitted for simplicity in FIG. 3.

Furthermore, the learning database 106 may store information related to known digital patterns indicative of objects in the image. For example, the learning database 106 may store patterns indicative of various abnormal (and normal) objects found in the breast. The database 106 may also store a set of basic training data (e.g., known abnormalities, etc.) to be used to train the deep learning algorithms. Of course, by utilizing more and more training data, the deep learning algorithms become more accurate over time. By pooling together medical image data (image or non-image based data) deep learning algorithms and other types of machine learning algorithms may be utilized to learn data patterns that can be used to not only detect and localize abnormalities, but also to understand normal variations among different individuals and populations.

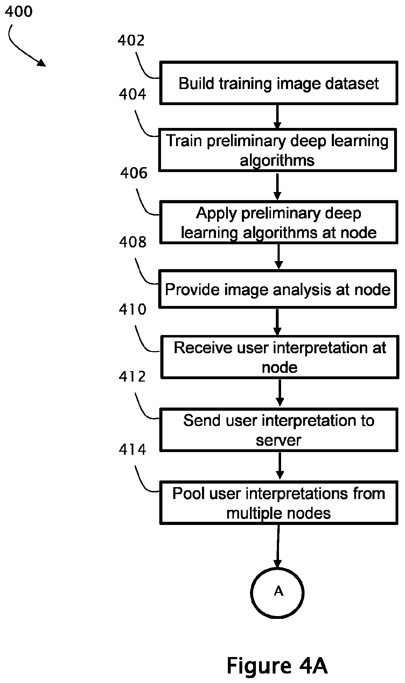

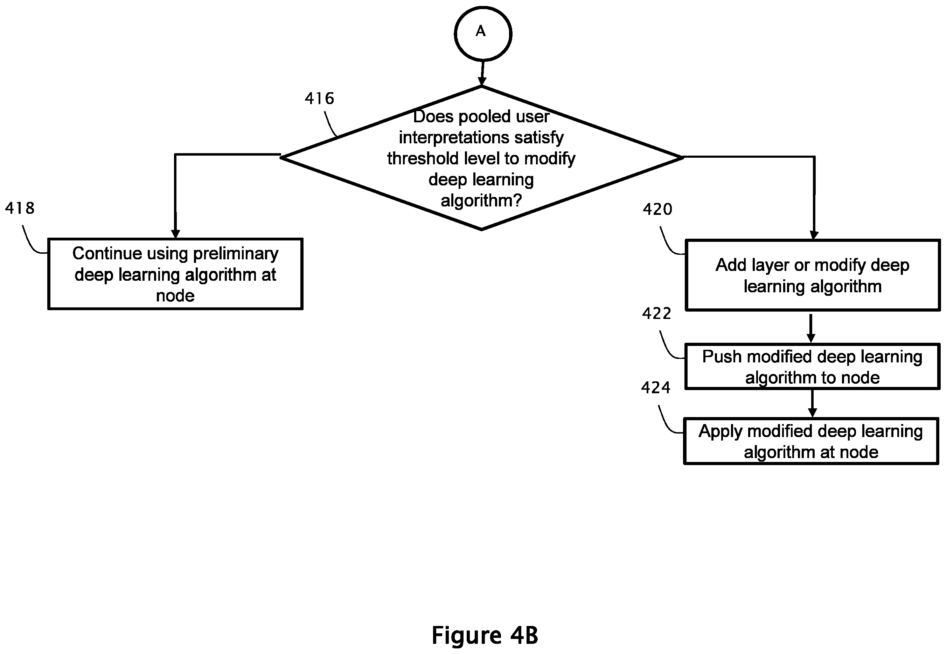

FIGS. 4A and 4B illustrate a flow diagram 400 provided to illustrate an exemplary process that may be performed in order to implement the dynamic self-learning medical image network system. At step 402, a training image dataset may be collected. For example, this initial image dataset may include a set of annotated medical images collected from a set of user experts. At step 404, this image dataset may be used to train a preliminary deep learning algorithm. At step 406, when a user 110a is viewing a new medical image, this preliminary deep learning algorithm may be applied. At step 408, an image analysis (indicating any detected abnormalities) may be provided at the node 102a. At step 410, user interaction data is received. For example, the user 110a may agree with the image analysis provided by the dynamic self-learning medical image network system, and indicate as much.

At step 412, the user interaction data may be sent from the node 102a to the server 150 through the central brain network 104. At step 414, the new user interaction data may be pooled with other user interpretation data related to a particular part of the deep learning algorithm. For example, the user interaction may pertain to a particular type of object being displayed at various nodes to various users. For example, a particular feature of an abnormal object may be specified through the user interaction. Each of these user interactions regarding the feature may be compiled together to determine whether users are identifying a particular feature, or else classifying it in a manner that is not presently encoded in the existing deep neural network or algorithm.

At step 416 it may be determined whether the pooled user interpretations satisfy a threshold level such that a modification to the preliminary deep learning algorithm is required. For example, the deep learning algorithm may only be modified, e.g., add a layer to the deep neural network, change a value, etc., if a predetermined threshold level is met. For example, to add a layer to the deep neural network, a confidence level, e.g., based on number of users providing the particular input, weight given to users, etc., of 98% may need to be met. Or, in another example, to change an existing value, a confidence level of 99.9% may need to be met.

At step 418, if the predetermined threshold level is not met, the system continues to use the preliminary deep learning algorithm. If, however, it is determined at step 416 that the threshold level is met, the deep learning algorithm may be modified (e.g., a new layer may be added). At step 422, the modified deep learning algorithm may be pushed to various nodes, and the modified deep learning algorithm may be applied to various images at the nodes (step 424).

It should be appreciated that in some embodiments, when the neural network is modified, it may need to be trained at the server with training data prior to pushing the algorithm to the various nodes. Thus, the above example is provided for illustrative purposes and should not be read as limiting.

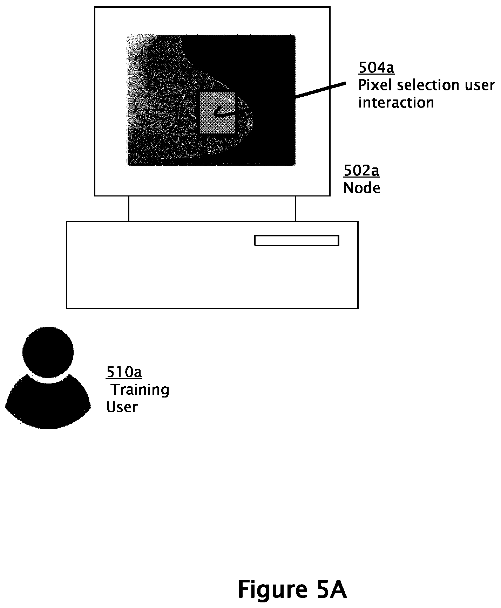

Referring now to FIGS. 5A-5H an exemplary process flow diagram illustrating various steps in implementing the dynamic self-learning medical image network system is shown. It should be appreciated that the following scenario focuses on user-based interactions at the nodes, but other embodiments may entail simply collecting data from the node without any user interaction at all. Thus, again, the following described process is provided for purposes of illustration, and should not be read as limiting.

Referring to FIG. 5A, a training user 510a may interact with one or more medical image slices at a node 502a. In one or more embodiments, the training user may be a user that is chosen to train the dynamic self-learning medical image network system. In order embodiments, the training user may be a volunteer. In yet another embodiment, there may be no distinction between training users and regular users, and all user interactions with the dynamic self-learning medical image network system may be given the same weight. As shown in FIG. 5A, the training user 510a may interact with the medical image by selecting a group of pixels (504a). For example, the selected group of pixels may represent an area of the medical image containing one or more abnormalities (or other objects of interest).

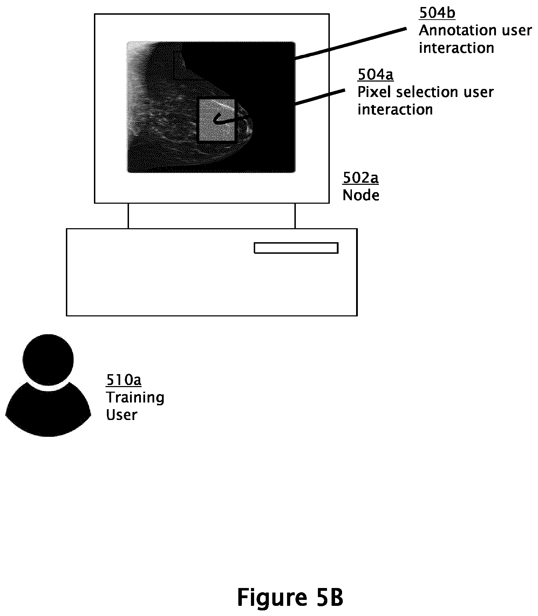

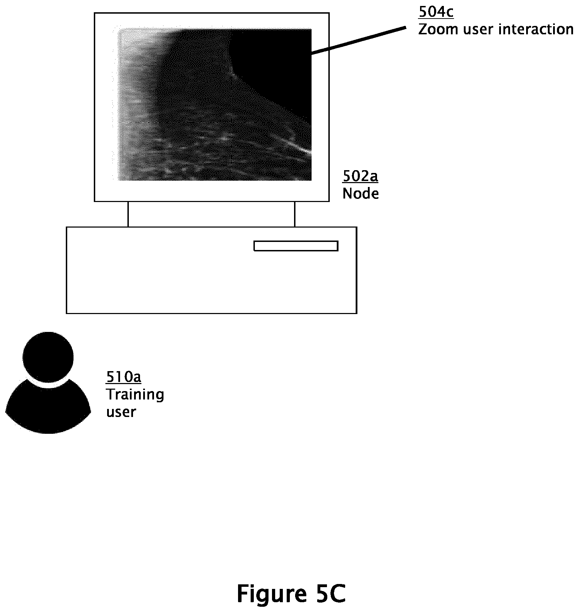

Referring now to FIG. 5B, the training user 510a may further annotate the one or more medical images (504b). In the illustrated embodiment, the user 510a may annotate the selected portion of the medical image to indicate that it pertains to a calcification object. FIG. 5C illustrates yet another user interaction 504c, where the system notes that the training user 510a zooms in on a particular area of the medical image.

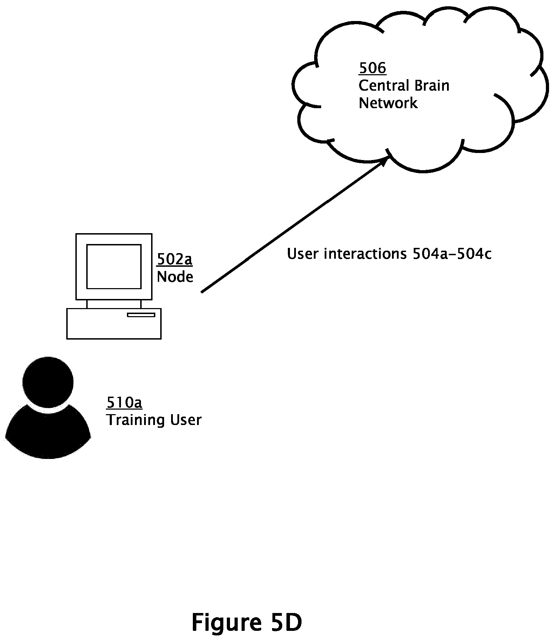

As shown in FIG. 5D, the user interactions 504a-504c are transmitted to the server 150 through the central brain network 506. It should be appreciated that in some embodiments, an instance of the dynamic self-learning medical image network system may reside at the node 502a itself. In other embodiments, the dynamic self-learning medical image network system may only be accessed through the central brain network 506.

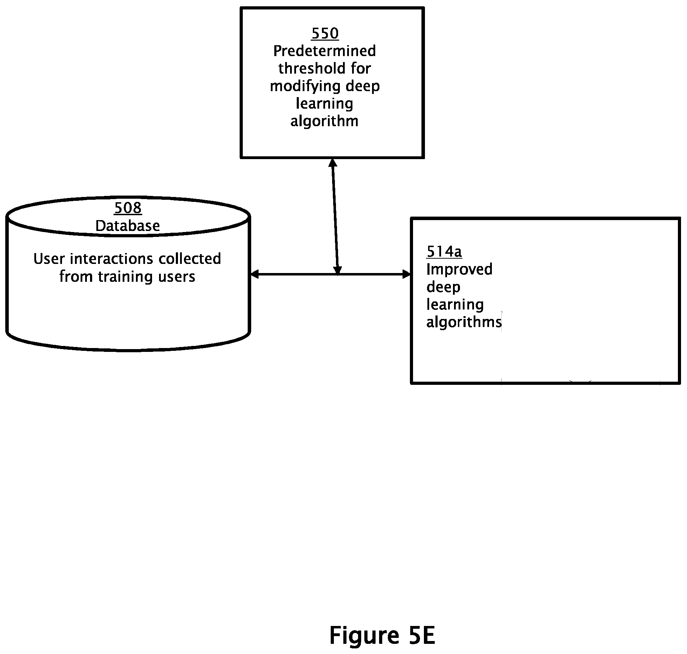

As shown in FIG. 5E, the user interactions 504a-504c may be stored in the database 508 associated with the dynamic self-learning medical image network system. This set of user interactions may be used to train the deep learning algorithms to result in a set of improved deep learning algorithms 514a. In the illustrated embodiment, the system consults with a predetermined threshold for modifying the deep learning algorithm 550 in order to determine whether the user input (e.g., pooled from various users) meets or exceeds the threshold. If the threshold is satisfied, the deep learning algorithm 514a is created. As discussed above, the improved deep learning algorithm 514a may be additional layers, modified values, or any other changes. It should be appreciated that this modification occurs without a need for re-programming of the neural network, and maybe done automatically by the dynamic self-learning medical image network system periodically whenever the threshold is met, in one or more embodiments.

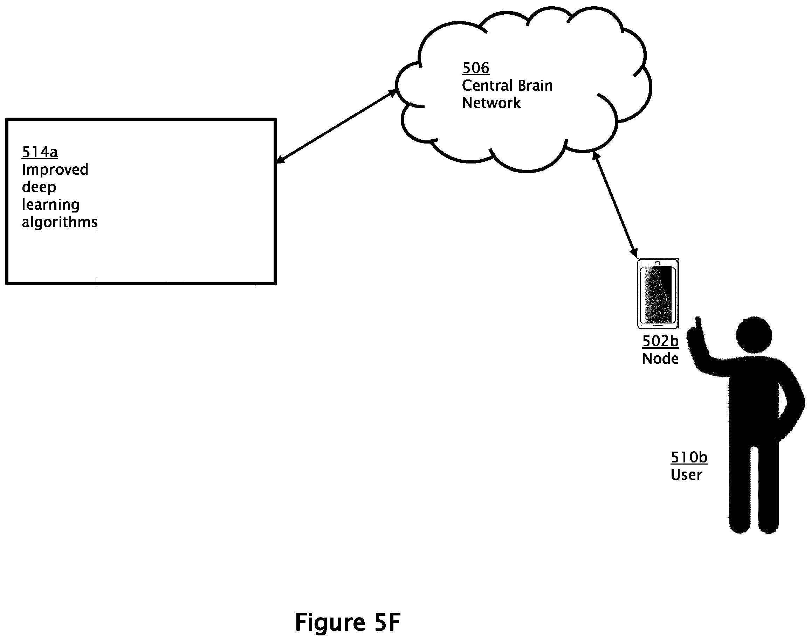

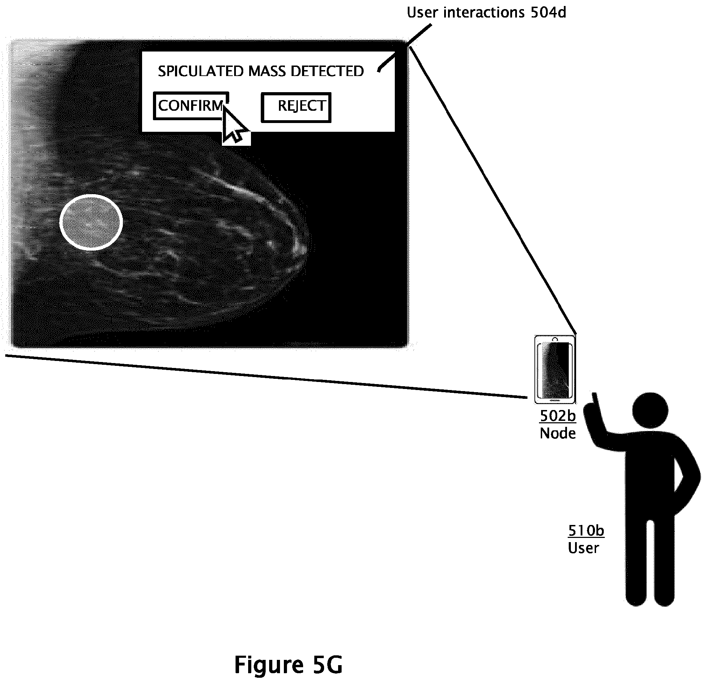

Referring now to FIG. 5F, the improved deep learning algorithm may be utilized at another node 502b, being accessed by another user 510b. In the illustrated embodiment, the other user 510b may be viewing a different set of medical images. As shown in FIG. 5G, the dynamic self-learning medical image network system may utilize the improved deep learning algorithm 514a to recognize one or more objects in the medical images being shown at node 502b. For example, a spiculated mass object may be detected, and the system may ask the user 510b to confirm or reject a recognized object. This user interaction 504d (e.g., confirm/reject) may be captured by the dynamic self-learning medical image network system.

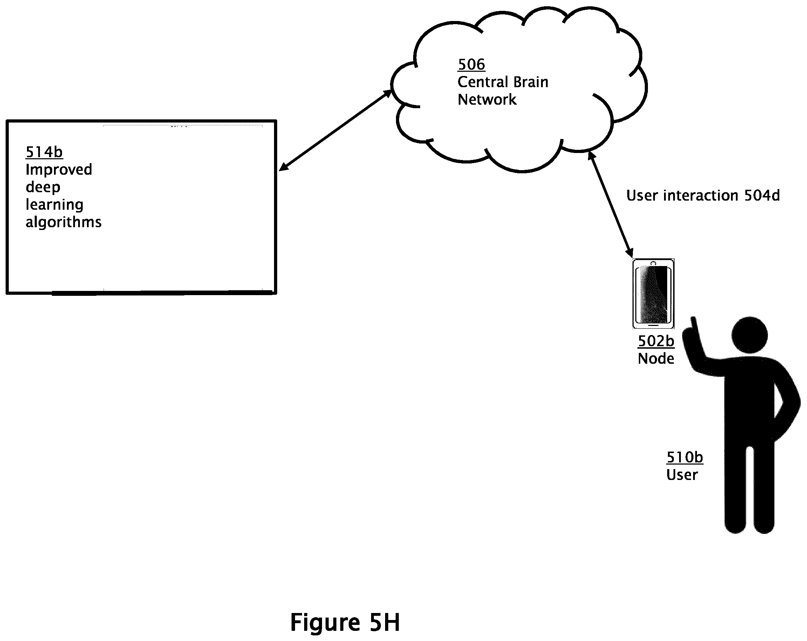

Finally, referring to FIG. 5H, user interaction 504d is used to improve the deep learning algorithms even further, thereby generating improved deep learning algorithms 514b. These improved deep learning algorithms 514b may be successfully used at other nodes to perform various analysis tasks. Thus, the dynamic self-learning medical image network system is greatly improved over time by receiving user interactions from a large number of users.

Having described exemplary embodiments of the dynamic self-learning medical image network system, it should be appreciated that the examples provided herein and depicted in the accompanying figures are only illustrative, and that other embodiments and examples also are encompassed within the scope of the appended claims. For example, while the flow diagrams provided in the accompanying figures are illustrative of exemplary steps; the overall image merge process may be achieved in a variety of manners using other data merge methods known in the art. The system block diagrams are similarly representative only, illustrating functional delineations that are not to be viewed as limiting requirements of the disclosed inventions. It will also be apparent to those skilled in the art that various changes and modifications may be made to the depicted and/or described embodiments, without departing from the scope of the disclosed inventions, which is to be defined only by the following claims and their equivalents. The specification and drawings are, accordingly, to be regarded in an illustrative rather than restrictive sense.

## Claims

1. A method for creating and using a dynamic self-learning medical image network system, the method comprising:
receiving, from a first node that displays one or more medical images to a user, initial user interaction data pertaining to one or more user interactions with the one or more initially obtained medical images;
training a deep learning algorithm based at least in part on the initial user interaction data received from the first node; and
wherein the initial user interaction data comprises an actual or estimated portion of at least one of the one or more medical images that was focused upon by at least one user, and/or a selection of one or more pixels associated with at least one of the one or more initially obtained medical images;
transmitting an instance of the trained deep learning algorithm to the first node and/or to one or more additional nodes, wherein at each respective node to which the instance of the trained deep learning algorithm is transmitted, the trained deep learning algorithm is applied to respective one or more subsequently obtained medical images in order to obtain a result; wherein the result comprises recognizing one or more objects in at least one of the one or more subsequently obtained medical images;
the method further comprising receiving, from the first node and/or one or more additional nodes, subsequent user interaction data pertaining to one or more subsequently obtained medical images, wherein the subsequent user interaction data is used to modify the trained deep learning algorithm.

2. The method of claim 1, wherein the initial user interaction data comprises an actual or estimated amount of time one or more users spent viewing one or more of the initially obtained medical images.

3. The method of claim 1 or 2, wherein the initial user interaction data comprises a description of a patient condition and/or written or recorded audio diagnostic findings, and/or at least one annotation on at least one of the one or more initially obtained medical images.

4. The method of any of claims 1-3, wherein the subsequent user interaction data is used to modify the trained deep learning algorithm if it is determined that the subsequent user interaction data satisfies a predetermined threshold confidence level indicating that the trained deep learning algorithm should be modified.

5. The method of any of claims 1-4, wherein the modification of the trained deep learning algorithm comprises adding one or more layers to the trained deep learning algorithm and/or modifying a respective structure of one or more existing layers of the trained deep learning algorithm.

6. A dynamic self-learning medical image network system, comprising: a plurality of nodes; and
a central brain server configured to
receive initial user interaction data from one or more nodes of the plurality, wherein the initial user interaction data pertains to one or more user interactions with one or more initially obtained medical images,
train a deep learning algorithm based at least in part on the initial user interaction data received from the first node, and
wherein the initial user interaction data comprises an actual or estimated portion of at least one of the one or more medical images that was focused upon by at least one user, and/or a selection of one or more pixels associated with at least one of the one or more initially obtained medical images;
transmit an instance of the trained deep learning algorithm to each node of the plurality,
wherein each node of the plurality is configured to apply the instance of the trained deep learning algorithm to one or more subsequently obtained medical images in order to obtain a result;
wherein the result comprises recognizing one or more objects in at least one of the one or more subsequently obtained medical images;
wherein the central brain server is configured to receive subsequent user interaction data from one or more nodes of the plurality pertaining to one or more subsequently obtained medical images, wherein the subsequent user interaction data is used to modify the trained deep learning algorithm.

7. The system of claim 6, wherein the initial user interaction data comprises an actual or estimated amount of time one or more users spent viewing one or more of the initially obtained medical images.

8. The system of claim 6 or 7, wherein the initial user interaction data comprises a description of a patient condition and/or written or recorded audio diagnostic findings, and/or at least one annotation on at least one of the one or more initially obtained medical images.

9. The system of any of claims 6-8, wherein the subsequent user interaction data is used to modify the trained deep learning algorithm if the subsequent user interaction data satisfies a predetermined threshold confidence level indicating that the trained deep learning algorithm should be modified.

10. The system of any of claims 6-9, wherein the central brain server modifies the trained deep learning algorithm by adding one or more layers to, and/or modifying a structure of one or more existing layers of, the trained deep learning algorithm.

## Patentansprüche

1. Verfahren zum Erzeugen und Verwenden eines dynamischen, selbstlernenden Netzwerksystems für medizinische Bilder, wobei das Verfahren Folgendes umfasst:
Empfangen, von einem ersten Knoten, der einem Benutzer ein oder mehrere medizinische Bilder anzeigt, von anfänglichen Benutzerinteraktionsdaten, die eine oder mehrere Benutzerinteraktionen mit dem einen oder den mehreren anfänglich erhaltenen medizinischen Bildern betreffen;
Trainieren eines Deep-Learning-Algorithmus mindestens teilweise basierend auf den von dem ersten Knoten empfangenen anfänglichen Benutzerinteraktionsdaten; und
wobei die anfänglichen Benutzerinteraktionsdaten einen tatsächlichen oder geschätzten Teilbereich mindestens eines des einen oder der mehreren medizinischen Bilder, auf den sich mindestens ein Benutzer konzentriert hat, und/oder eine Auswahl eines oder mehrerer Pixel, die mindestens einem des einen oder der mehreren anfänglich erhaltenen medizinischen Bilder zugeordnet sind, umfassen;
Übertragen einer Instanz des trainierten Deep-Learning-Algorithmus an den ersten Knoten und/oder an einen oder mehrere zusätzliche Knoten, wobei an jedem jeweiligen Knoten, an den die Instanz des trainierten Deep-Learning-Algorithmus übertragen wird, der trainierte Deep-Learning-Algorithmus auf jeweils ein oder mehrere nachfolgend erhaltene medizinische Bilder angewendet wird, um ein Ergebnis zu erhalten; wobei das Ergebnis Erkennen eines oder mehrerer Objekte in mindestens einem des einen oder der mehreren nachfolgend erhaltenen medizinischen Bilder umfasst;
wobei das Verfahren ferner Empfangen, von dem ersten Knoten und/oder einem oder mehreren zusätzlichen Knoten, von nachfolgenden Benutzerinteraktionsdaten, die ein oder mehrere nachfolgend erhaltene medizinische Bilder betreffen, umfasst, wobei die nachfolgenden Benutzerinteraktionsdaten verwendet werden, um den trainierten Deep-Learning-Algorithmus zu modifizieren.

2. Verfahren nach Anspruch 1, wobei die anfänglichen Benutzerinteraktionsdaten eine tatsächliche oder geschätzte Zeitdauer umfassen, die ein oder mehrere Benutzer mit dem Betrachten eines oder mehrerer der anfänglich erhaltenen medizinischen Bilder verbracht haben.

3. Verfahren nach Anspruch 1 oder 2, wobei die anfänglichen Benutzerinteraktionsdaten eine Beschreibung eines Patientenzustands und/oder schriftliche oder als Audioaufzeichnung vorliegende diagnostische Befunde und/oder mindestens eine Annotation auf mindestens einem des einen oder der mehreren anfänglich erhaltenen medizinischen Bilder umfassen.

4. Verfahren nach einem der Ansprüche 1 bis 3, wobei die nachfolgenden Benutzerinteraktionsdaten verwendet werden, um den trainierten Deep-Learning-Algorithmus zu modifizieren, wenn bestimmt wird, dass die nachfolgenden Benutzerinteraktionsdaten einen vorgegebenen Konfidenzschwellenwert erfüllen, der angibt, dass der trainierte Deep-Learning-Algorithmus zu modifizieren ist.

5. Verfahren nach einem der Ansprüche 1 bis 4, wobei die Modifizierung des trainierten Deep-Learning-Algorithmus Hinzufügen einer oder mehrerer Schichten zu dem trainierten Deep-Learning-Algorithmus und/oder Modifizieren einer jeweiligen Struktur einer oder mehrerer bestehender Schichten des trainierten Deep-Learning-Algorithmus umfasst.

6. Dynamisches, selbstlernendes Netzwerksystem für medizinische Bilder, das Folgendes umfasst: eine Vielzahl von Knoten; und
einen zentralen Gehirnserver, der zu Folgendem ausgelegt ist:
Empfangen anfänglicher Benutzerinteraktionsdaten von einem oder mehreren Knoten der Vielzahl, wobei die anfänglichen Benutzerinteraktionsdaten eine oder mehrere Benutzerinteraktionen mit einem oder mehreren anfänglich erhaltenen medizinischen Bildern betreffen,
Trainieren eines Deep-Learning-Algorithmus mindestens teilweise basierend auf den von dem ersten Knoten empfangenen anfänglichen Benutzerinteraktionsdaten, und wobei die anfänglichen Benutzerinteraktionsdaten einen tatsächlichen oder geschätzten Teilbereich mindestens eines des einen oder der mehreren medizinischen Bilder, auf den sich mindestens ein Benutzer konzentriert hat, und/oder eine Auswahl eines oder mehrerer Pixel, die mindestens einem des einen oder der mehreren anfänglich erhaltenen medizinischen Bilder zugeordnet sind, umfassen;
Übertragen einer Instanz des trainierten Deep-Learning-Algorithmus an jeden Knoten der Vielzahl,
wobei jeder Knoten der Vielzahl dazu ausgelegt ist, die Instanz des trainierten Deep-Learning-Algorithmus auf ein oder mehrere nachfolgend erhaltene medizinische Bilder anzuwenden, um ein Ergebnis zu erhalten;
wobei das Ergebnis Erkennen eines oder mehrerer Objekte in mindestens einem des einen oder der mehreren nachfolgend erhaltenen medizinischen Bilder umfasst;
wobei der zentrale Gehirnserver dazu ausgelegt ist, nachfolgende Benutzerinteraktionsdaten von einem oder mehreren Knoten der Vielzahl, die ein oder mehrere nachfolgend erhaltene medizinische Bilder betreffen, zu empfangen, wobei die nachfolgenden Benutzerinteraktionsdaten verwendet werden, um den trainierten Deep-Learning-Algorithmus zu modifizieren.

7. System nach Anspruch 6, wobei die anfänglichen Benutzerinteraktionsdaten eine tatsächliche oder geschätzte Zeitdauer umfassen, die ein oder mehrere Benutzer mit dem Betrachten eines oder mehrerer der anfänglich erhaltenen medizinischen Bilder verbracht haben.

8. System nach Anspruch 6 oder 7, wobei die anfänglichen Benutzerinteraktionsdaten eine Beschreibung eines Patientenzustands und/oder schriftliche oder als Audioaufzeichnung vorliegende diagnostische Befunde und/oder mindestens eine Annotation auf mindestens einem des einen oder der mehreren anfänglich erhaltenen medizinischen Bilder umfassen.

9. System nach einem der Ansprüche 6 bis 8, wobei die nachfolgenden Benutzerinteraktionsdaten verwendet werden, um den trainierten Deep-Learning-Algorithmus zu modifizieren, wenn die nachfolgenden Benutzerinteraktionsdaten einen vorgegebenen Konfidenzschwellenwert erfüllen, der angibt, dass der trainierte Deep-Learning-Algorithmus zu modifizieren ist.

10. System nach einem der Ansprüche 6 bis 9, wobei der zentrale Gehirnserver den trainierten Deep-Learning-Algorithmus durch Hinzufügen einer oder mehrerer Schichten zu dem trainierten Deep-Learning-Algorithmus und/oder Modifizieren einer Struktur einer oder mehrerer bestehender Schichten des trainierten Deep-Learning-Algorithmus modifiziert.

## Revendications

1. Procédé de création et d'utilisation d'un système de réseau d'images médicales auto-apprenant dynamique, le procédé comprenant :
la réception, en provenance d'un premier nœud qui affiche une ou plusieurs images médicales à un utilisateur, de données d'interaction utilisateur initiales relatives à une ou plusieurs interactions utilisateur avec les une ou plusieurs images médicales initialement obtenues ;
l'entraînement d'un algorithme d'apprentissage profond sur la base au moins en partie des données d'interaction utilisateur initiales reçues du premier nœud ; et
les données d'interaction utilisateur initiales comprenant une partie réelle ou estimée d'au moins l'une des une ou plusieurs images médicales qui a retenu l'attention d'au moins un utilisateur, et/ou une sélection d'un ou plusieurs pixels associés à au moins l'une des une ou plusieurs images médicales initialement obtenues ;
la transmission d'une instance de l'algorithme d'apprentissage profond entrainé au premier nœud et/ou à un ou plusieurs nœuds supplémentaires, où, au niveau de chaque nœud respectif auquel est transmise l'instance de l'algorithme d'apprentissage profond entrainé, l'algorithme d'apprentissage profond entrainé est appliqué à une ou plusieurs images médicales respectives obtenues ultérieurement afin d'obtenir un résultat ; le résultat comprenant la reconnaissance d'un ou plusieurs objets dans au moins l'une des une ou plusieurs images médicales obtenues ultérieurement ;
le procédé comprenant en outre la réception, en provenance du premier nœud et/ou d'un ou plusieurs nœuds supplémentaires, de données d'interaction utilisateur ultérieures relatives à une ou plusieurs images médicales obtenues ultérieurement, les données d'interaction utilisateur ultérieures étant utilisées pour modifier l'algorithme d'apprentissage profond entraîné.

2. Procédé selon la revendication 1, dans lequel les données d'interaction utilisateur initiales comprennent une quantité réelle ou estimée de temps passé par un ou plusieurs utilisateurs à visualiser une ou plusieurs des images médicales initialement obtenues.

3. Procédé selon la revendication 1 ou 2, dans lequel les données d'interaction utilisateur initiales comprennent une description d'un état de patient et/ou des résultats de diagnostic consignés par écrit ou sous forme d'enregistrement audio, et/ou au moins une annotation sur au moins l'une des une ou plusieurs images médicales initialement obtenues.

4. Procédé selon l'une quelconque des revendications 1 à 3, dans lequel les données d'interaction utilisateur ultérieures sont utilisées pour modifier l'algorithme d'apprentissage profond entraîné s'il est déterminé que les données d'interaction utilisateur ultérieures satisfont à un niveau de confiance seuil prédéterminé indiquant que l'algorithme d'apprentissage profond entraîné doit être modifié.

5. Procédé selon l'une quelconque des revendications 1 à 4, dans lequel la modification de l'algorithme d'apprentissage profond entraîné comprend l'ajout d'une ou plusieurs couches à l'algorithme d'apprentissage profond entraîné et/ou la modification d'une structure respective d'une ou plusieurs couches existantes de l'algorithme d'apprentissage profond entraîné.

6. Système de réseau d'images médicales auto-apprenant dynamique, comprenant : une pluralité de nœuds ; et
un serveur formant cerveau central configuré pour recevoir des données d'interaction utilisateur initiales en provenance d'un ou plusieurs nœuds de la pluralité, les données d'interaction utilisateur initiales étant relatives à une ou plusieurs interactions utilisateur avec les une ou plusieurs images médicales initialement obtenues,
entraîner un algorithme d'apprentissage profond sur la base au moins en partie des données d'interaction utilisateur initiales reçues du premier nœud, et
les données d'interaction utilisateur initiales comprenant une partie réelle ou estimée d'au moins l'une des une ou plusieurs images médicales qui a retenu l'attention d'au moins un utilisateur, et/ou une sélection d'un ou plusieurs pixels associés à au moins l'une des une ou plusieurs images médicales initialement obtenues ;
transmettre à chaque nœud de la pluralité une instance de l'algorithme d'apprentissage profond entrainé,
chaque nœud de la pluralité étant configuré pour appliquer l'instance de l'algorithme d'apprentissage profond entrainé à une ou plusieurs images médicales obtenues ultérieurement afin d'obtenir un résultat ;
le résultat comprenant la reconnaissance d'un ou plusieurs objets dans au moins l'une des une ou plusieurs images médicales obtenues ultérieurement ;
le serveur formant cerveau central étant configuré pour recevoir des données d'interaction utilisateur ultérieures provenant d'un ou plusieurs nœuds de la pluralité relatives à une ou plusieurs images médicales obtenues ultérieurement, les données d'interaction utilisateur ultérieures étant utilisées pour modifier l'algorithme d'apprentissage profond entraîné.

7. Système selon la revendication 6, dans lequel les données d'interaction utilisateur initiales comprennent une quantité réelle ou estimée de temps passé par un ou plusieurs utilisateurs à visualiser une ou plusieurs des images médicales initialement obtenues.

8. Système selon la revendication 6 ou 7, dans lequel les données d'interaction utilisateur initiales comprennent une description d'un état de patient et/ou des résultats de diagnostic consignés par écrit ou sous forme d'enregistrement audio, et/ou au moins une annotation sur au moins l'une des une ou plusieurs images médicales initialement obtenues.

9. Système selon l'une quelconque des revendications 6 à 8, dans lequel les données d'interaction utilisateur ultérieures sont utilisées pour modifier l'algorithme d'apprentissage profond entraîné si les données d'interaction utilisateur ultérieures satisfont à un niveau de confiance seuil prédéterminé indiquant que l'algorithme d'apprentissage profond entraîné doit être modifié.

10. Système selon l'une quelconque des revendications 6 à 9, dans lequel le serveur formant cerveau central modifie l'algorithme d'apprentissage profond entraîné en ajoutant une ou plusieurs couches à, et/ou en modifiant une structure d'une ou plusieurs couches existantes de, l'algorithme d'apprentissage profond entraîné.
